(19) Europäisches Patentamt
European Patent Office
Office européen des brevets

(11) Publication number: **0 020 401**
**B1**

(12) # EUROPEAN PATENT SPECIFICATION

(45) Date of publication of patent specification: **09.02.83**

(51) Int. Cl.³: **C 07 D 241/08,**
C 07 D 243/08,
C 07 D 241/38 //C08K5/34

(21) Application number: **79901124.2**

(22) Date of filing: **01.08.79**

(86) International application number:
**PCT/US79/00563**

(87) International publication number:
**WO 80/00702 17.04.80 Gazette 80/8**

(54) **IMPROVED PROCESS FOR THE SYNTHESIS OF 1,4-DIAZA-2-CYCLOALKANONE AND SUBSTITUTED DERIVATIVES THEREOF.**

(30) Priority: **21.09.78 US 944478**

(43) Date of publication of application:
**07.01.81 Bulletin 81/1**

(45) Publication of the grant of the patent:
**09.02.83 Bulletin 83/6**

(84) Designated Contracting States:
**CH DE FR GB NL SE**

(56) References cited:
**EP - A - 0 001 284**
**EP - A - 0 001 559**
**EP - A - 0 004 460**
**FR - A - 1 179 978**
**US - A - 3 992 432**
**US - A - 4 167 512**

**ANGEWANDTE CHEMIE, vol. 16, nr. 8, August 1977, International Edition in English; Verlag Chemie GmbH, Weinheim, New York E. DEHMLOW "Advances in Phase-Transfer Catalysis". pages 493—505**

(73) Proprietor: **The B.F. GOODRICH Company**
**Dept. 0015 WHB-6 500 South Main Street**
**Akron, Ohio 44318 (US)**

(72) Inventor: **LAI, John, Ta-Yuan**
**663 Tollis Parkway**
**Broadview Heights, OH 44147 (US)**

(74) Representative: **von Kreisler, Alek, Dipl.-Chem. et al,**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

# Improved process for the synthesis of 1,4-diaza-2-cycloalkanone and substituted derivatives thereof

## Background of the invention

### Field of the invention

This invention is directed to the synthesis of 1,4-diaza-cycloalkan-2-ones and substituted derivatives thereof by a technique involving solid-liquid phase transfer phenomena.

### Description of the prior art

The increasing use of polymers in place of the more traditional types of structural materials (e.g. wood, metals, etc.) has necessitated the compounding of such polymers with a variety of stabilizers in order to enhance the ability of such polymers to withstand prolonged exposure to a variety of degradative forces. Degradation of such environmentally sensitive polymers can be caused by exposure to light, heat and/or air. Such degradation is usually manifest by either a partial or total loss of structural integrity, changes in light transmission properties, changes in color, loss or reduction in flexibility and/or resiliency, or any combination of the above phenomenon. As will be appreciated, the stabilizers which are used in conjunction with the above polymeric materials, in addition to providing protection against such degradative changes, must also be compatible with the aesthetic properties of the polymeric article and be effective at low concentrations. The economics of the marketplace dictate that these stabilizers be relatively inexpensive and capable of preparation from readily available starting materials by simple and straightforward synthesis techniques.

The diazacycloalkanones have been found to be highly effective in the stabilization of polymeric materials against the photodegradative forces of ultraviolet light. The efficacy of such materials in the UV stabilization of polymers is described in copending European Patent Applications 78 100 942.8 (EP—A—0001284) and 78 100 941.0 (EP—A—0001560) entitled "Substituted 2-keto-1,4-Diaza-Cycloalkanes and UV Light Stabilized Compositions Containing Same" and "UV Light Stabilized Compositions Containing Substituted 1,5-diazacyclo Alkanes and Novel Compounds". The aforementioned applications disclose methods for the preparation of compounds useful in the stabilization of UV sensitive polymer compositions. European application 78 100 940.2 (EP—A—0001559) entitled "Synthesis of 2-keto-1,4-Diazacycloalkanes" discloses a variety of techniques for the convenient synthesis of highly effective UV stabilizer compounds. The principal advantage of the synthesis described in European application 78 100 940.2 (EP—A—0001559) involves the utilization of readily available starting materials, conventional processing apparatus, the absence of hydrogen cyanide and the attendant hazards associated therewith.

In application 78 100 940.2 (EP—A—0001559) in the section entitled "Background of the Invention", certain references are enumerated which disclose cycloalkanes as useful UV stabilizers—see for example OLS 2,315,042; JAP PATS. 74-53,571 and 74-53.572; and U.S. Patents 3,919,234; 3,920,659 and 3,928,330. Also discussed in this same Section are structurally similar compounds and the limitations on the synthesis of such compounds and the analogs thereof. To the extent that this discussion is relevant to subject matter of the invention described and claimed hereinafter, it is hereby incorporated by reference in its entirety.

As noted in copending application 78 100 940.2 (EP—A—0001559) prior art processes discussed therein do not describe convenient techniques for the preparation of poly-substituted 1,4-diazacycloalkanones. The limitations apparent in such processes are most pronounced where one attempts to synthesize symmetrically substituted compounds. Other aspects of the prior art processes in need of improvement involve (a) reduction in size of reaction mass, (b) simplification of work-up procedure for separation of product from reaction mass, and (c) reduction in reaction time. To the extent that such deficiencies in the prior art can be remedied, synthesis of the desired product will be accomplished more efficiently and more economically.

1,4-Diazacycloalkan-2-ones are known from U.S. Patent 4 190 571.

Accordingly, it is the object of this invention to remedy the above as well as related deficiencies in the prior art and more specifically, it is the principal object of this invention to provide a more efficient and economical process for the synthesis of 1,4-diaza-cycloalkanones, especially for the synthesis of symmetrically substituted 1,4-diazacycloalkanones, and from readily available starting materials.

Additional objects of this invention include providing an improved process for the synthesis of symmetrically substituted 1,4-diaza-cycloalkan-2-ones without the risks attendant in the use of hydrogen cyanide.

The above and related objects are achieved by contacting an appropriately substituted diamine with co-reactants in the presence of a phase transfer catalyst and solid caustic. In one of the preferred embodiments of this invention, the exotherm of the reaction is controlled by chilling of the reaction vessel. In another of the preferred embodiments of the invention, the reactants and catalysts are contacted with one another while dispersed in a nonpolar organic solvent, such as dichloromethane in which the product is highly soluble.

Description of the invention including preferred embodiments

In a typical embodiment of the process of this invention, an appropriately substituted diamine and as a co-reactant, an $\alpha$-trihalo (e.g. chloro) alkyl alcohol, a mixture of a haloform and an $\alpha$-cyanoalkyl alcohol, or a mixture of a haloform and an aliphatic ketone, in the desired relative concentrations, are introduced into a reaction vessel followed thereafter by the addition to said vessel of a phase transformation catalyst dissolved in a non-polar organic solvent. To this reaction mass is thereafter added solid caustic, such as solid sodium hydroxide. The reaction vessel is preferably at least partially immersed in an ice bath to control the exotherm of the reaction. The presence of the solid caustic in the reaction medium also serves a similar function that is, it retards the rate of reaction and thus the heat generated during the combination of the reactants is reduced significantly.

The ratio of reactants to one another in this process is not believed to be critical to the formation of its desired product. However, where one desires to obtain high yields and ease of separation of the reaction product from the various reactants and catalysts used in such preparation, it is preferable to adjust the relative mole concentration of materials so that the ratio of diamine to co-reactant (e.g. $\alpha$-trihaloalkylalcohol) is in the range of from 0.5:1 to 1:1.

As the result of the interaction of the above materials in the reaction vessel, a water-soluble solid is formed. This solid can be removed from the nonpolar reaction medium by filtration or by the addition of water to the reaction medium followed thereafter by separation of the aqueous phase from the organic fluid phase. The aqueous phase is thereafter further extracted with an organic solvent such as chloroform and the combined organic solutions washed with water, dried over sodium sulphate and concentrated. The desired product is crystallized or distilled from the combined organic solutions and recrystallized in the conventional fashion to yield a relatively pure product.

The substituted diamines suitable for use in this process have the following structural formula:

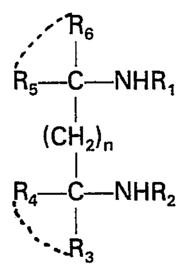

wherein $R_1$ and $R_2$ are independently selected from hydrogen, halogen, alkyl of 1 to 12 carbon atoms, hydroxyalkyl, haloalkyl, cyanoalkyl, aminoalkyl, iminoalkyl, alkyloxyalkylene, nitro, hydroxyalkyloxyalkylene, cyanoalkyloxyalkylene, alkenyl, alkynyl and carboalkoxy; $R_3$ through $R_6$ are independently selected from alkyl of 1 to 12 carbon atoms, cycloalkyl, hydroxycycloalkyl, aminoalkyl, iminoalkyl, alkenyl and aralkyl provided further that any two of said substituents pendant from the same carbon atom can be hydrogen atoms or collectively form a cyclic or alicyclic hydrocarbon; and n is 0 to 3.

Compounds within the scope of the above structural formula which are especially suitable for use in the process of this invention are the N′-alkyl-2-alkyl-1,2-alkane diamines, e.g. N′-isopropyl-2-methyl-1,2-propanediamine. Additional 1,2-diamines which are highly effective for use in the process of this invention include N′-propyl-2-methyl-1,2-propane-diamine, N′-t-butyl-2-methyl-1,2-propanediamine, N′-t-octyl-2-methyl-1,2-propanediamine, ethylene-2,2-bis-"N"-(2-methyl-1,2-propanediamine), and 2,5-dimethylhexane-2,5-bis-'N'-(2-methyl-1,2-propanediamine).

The co-reactants which are suitable for use in the process of this invention are $\alpha$-trihaloalkyl alcohols, mixtures of a haloform and an $\alpha$-cyanoalkyl alcohols, and/or mixtures of a haloform and an aliphatic ketones. The trihaloalkylalcohol which is suitable for use in the process of this invention is represented by the following formulae

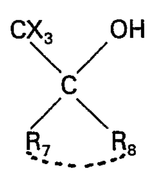

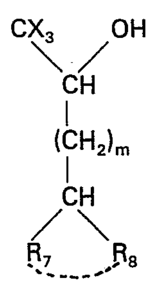

wherein $R_7$ and $R_8$ are selected from the same group of substituents as $R_3$ through $R_6$ above; X is halogen; and m is 0 to 4.

In the preferred embodiments of the process of this invention, the $\alpha$-trihaloalkyl alcohol is

symmetrically substituted at the $\beta$ carbon. One such material which is especially preferred for use in the process of this invention is 1,1,1-trichloro-2-methyl-2-propanol hydrate. The alcohols which are highly suitable for use in this process include $\alpha$-trichloromethyl-2-propanol, $\alpha$-trichloromethyl-cyclohexanol, and $\alpha$-trichloromethyl-1-butanol.

The co-reactant mixtures which are suitable for use in the process of this invention contain a haloform, (e.g. chloroform or bromoform) in addition to an $\alpha$-cyanoalkylalcohol or an aliphatic ketone. The concentration of the haloform relative to the other component of the mixture can vary and is preferably present in stoichiometric amounts (in relation to the $\alpha$-cyanoalkylalcohol, at a mole ratio of 1:2 and, in relation to the aliphatic ketone, at a mole ratio of 1:1) although a stoichiometric excess is most preferred.

The $\alpha$-cyanoalkylalcohols which are suitable for use in this process can be represented by the following formulae

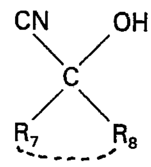 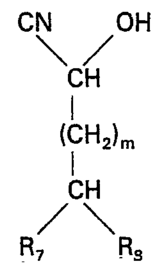

wherein $R_7$ and $R_8$ are selected from the same group of substituents as $R_3$ through $R_6$ above; and m is 0 to 4.

The aliphatic ketones which are suitable for use in the process of this invention are represented by the following formula

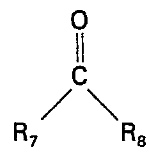

wherein $R_7$ and $R_8$ are selected from the same group of substituents as $R_3$ through $R_6$ above.

One or more of above co-reactants, as indicated previously, are contacted with the diamine in the presence of a "phase transfer catalysts". The phrase "phase transfer catalysts" is intended to describe, in the context of this invention, any compound, which in the presence of the above reactants, causes a condensation of the co-reactants and the diamine in such a fashion so as to result in the formation of a cyclic ketone wherein the oxygen is doubly bonded to an $\alpha$-carbon of the co-reactant material.

Materials which have been found to effectively catalyze condensation of the diamine and co-reactants in this fashion can be represented by the following formula:

$$Q^+B^-$$

wherein $Q^+$ is $NH_nR_x$, $PH_nR_x$ provided that

R is alkyl of 1—20 carbon atoms, aryl or aralkyl;

n is 0—4 and x is 0—4 provided further that the sum of n plus x equals 4;

$B^-$ is a monovalent anion, such as a halide, acetate, perchlorate, sulfate, or hydrosulfate. Catalyst compositions which are preferred for the process of this invention include cetyl trimethylammonium bromide, methyl tricaprylylammonium chloride, cetyl tributyl phosphonium bromide, tetrabutylammonium hydrogen sulfate and tetrabutylammonium chloride.

In addition to the above reactants and catalysts, a stoichiometric excess of solid caustic is also employed in the process of this invention. This caustic material is added in solid form to the reaction mass preferably subsequent to the addition to the reactor of all reactants and catalyst. The concentrations of caustic relative to diamine used in this process can vary and will ordinarily be present in the reaction mass in excess of stoichiometric quantities; preferably at the relative mole ratio of 3.3:1 to 6:1. The addition of the solid caustic material in lieu of caustic solution to the reaction mass (a) reduces the size of the reaction mass, (b) simplifies the separation of the desired product from the reaction mass, and (e) enhances the overall efficiency of the synthesis, thereby reducing the reaction time.

The 1,4-diaza-cycloalkan-2-ones produced in accord with the above procedures are highly effective at low concentrations in the stabilization of UV degradable polymeric materials.

Examples

The Examples which follow further define, describe and illustrate the process of this invention. Apparatus and techniques used in such process are standard or as hereinbefore described. Products and percentages appearing in such Examples are by weight unless otherwise stipulated.

Example I

N'-isopropyl-2-methyl-1,2-propane diamine (13.0 g, 0.1 mols) and 2-trichloromethyl-2-propanol hydrate (23.1 g, 0.13 mol) were placed in a 3 neck flask which itself was immersed in an icebath. 50 milliliters dichloromethane and benzyltriethyl ammonium chloride (0.91 g, 0.004 mol) were then added to the flask, followed by the metered addition of solid sodium hydroxide (a total of 48 g or 0.6 mols); the rate of addition being adjusted so as to maintain the reaction temperature below about 10°C. A few hours after the addition of caustic, the reaction was complete, as confirmed by gas chromatography. Water was added to the flask until all salts were dissolved. The two layers, which are formed in the flask, are separated and the aqueous phase extracted once with chloroform. The combined organic solutions were then washed three times with 50 milliliters water, dried over sodium sulfate and concentrated. The solid which is formed in the flask was recrystallized from pentane to obtain 9.5 grams of needlelike crystals. The ir spectrum and elemental analysis were consistent with the structure of the desired product.

Example II

The procedures of Example I are repeated except with the following combination of reactants.

| Example No. | 1,2-diamine | Co-reactant |
|---|---|---|
| II | N'-propyl-2-methyl-1,2-propane diamine | 2-trichloromethyl-2-propanol hydrate |
| III | N'-butyl-4-methyl-2,4-pentane diamine | 2-trichloromethyl-2-propanol hydrate |
| IV | N,N'-dimethyl-ethylene diamine | 2-trichloromethyl-2-propanol hydrate |
| V | N'-isopropyl-2-methyl-1,2-propane diamine | acetone cyanohydrin (0.12 moles) chloroform (0.22 moles) |
| VI | N'-isopropyl-2-methyl-1,2-propane diamine | cyclohexanone cyanohydrin chloroform |
| VII | 1,2-ethane-bis-N'-(2-methyl-1,2-propane diamine) | acetone cyanohydrin chloroform |

**Claims**

1. A process for the synthesis of 1,4-diaza-cycloalkan-2-ones, characterized by contacting, in the presence of a phase transfer catalyst, which in the presence of the following reactants, causes a condensation of the co-reactants and the diamine in such a fashion so as to result in the formation of a cyclic ketone wherein the oxygen is doubly bonded to an $\alpha$-carbon of the co-reactant material, a diamine of the formula

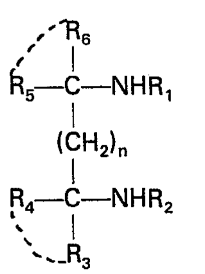

wherein

$R_1$ and $R_2$ are independently selected from hydrogen, halogen, alkyl of 1 to 12 carbon atoms, hydroxyalkyl, haloalkyl, cyanoalkyl, aminoalkyl, iminoalkyl, alkyloxyalkylene, nitro, hydroxyalkyloxyalkylene, cyanoalkyloxyalkylene, alkenyl, alkynyl and carboalkoxy; $R_3$ through $R_6$

being independently selected from alkyl of 1 to 12 carbon atoms, cycloalkyl, hydroxycycloalkyl, aminoalkyl, iminoalkyl, alkenyl, and aralkyl,

provided further that any two of the above substituents pendant from the same carbon atom can be hydrogen atoms or collectively form a cyclic or alicyclic hydrocarbon; and n is a number from 0 to 3;

with a stoichiometric excess of both (i) solid caustic and (ii) an $\alpha$-trihaloalkylalcohol of the formulae

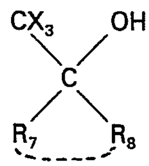
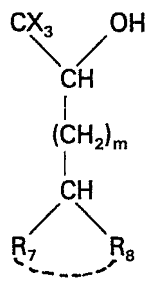

wherein $R_7$ and $R_8$ are the same as $R_3$—$R_6$ defined hereinabove, m is 0 to 4 and X is halogen.

2. The process of Claim 1 wherein the temperature of the reactants is maintained at 10°C or below.

3. The process of Claim 1, wherein the phase transfer catalyst is an ammonium salt.

4. The process of Claim 1 wherein the reactants and catalyst are combined in a nonpolar organic reaction medium.

5. The process of Claim 4, wherein the 1,4-diaza-cycloalkan-2-one is highly soluble in the nonpolar organic reaction medium.

6. The process of Claim 1 wherein the diamine is N'-isopropyl-2-methyl-1,2-propanediamine.

7. The process of Claim 1 wherein the alcohol is an $\alpha$-trichloroalkylalcohol.

8. The process of Claim 1 wherein the relative mole concentration of solid caustic to the diamine is in the range of from 3.3:1 to 6:1.

9. The process of Claim 1 wherein the solid caustic is sodium hydroxide.

10. A modification of the process of claim 1, characterized in that in place of (ii) a mixture of haloform and an $\alpha$-cyanoalkylalcohol of the formulae

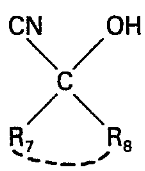
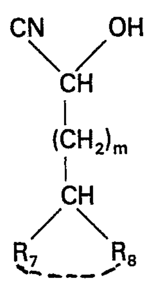

wherein $R_7$ and $R_8$ are the same as $R_3$—$R_6$ defined herein above and m is 0 to 4, is used.

11. A modification of the process of claim 1, characterized in that in place of (ii) a mixture of haloform and an aliphatic ketone of the formula:

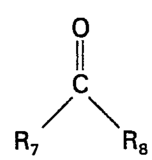

wherein $R_7$ and $R_8$ are the same as $R_3$—$R_6$ defined hereinabove is used.

**Patentansprüche**

1. Verfahren zur Synthese von 1,4-Diaza-cycloalkan-2-onen, dadurch gekennzeichnet, daß in Gegenwart eines Phasen-Transfer-Katalysators, der in Anwesenheit der folgenden Reaktionsteilnehmer eine Kondensation der Co-Reaktionspartner und des Diamins in solcher Weise herbeiführt, daß ein cyclisches Keton gebildet wird, in dem der Sauerstoff an den $\alpha$-Kohlenstoff des Co-Reaktionspartner-Materials gebunden ist, ein Diamin der Formel

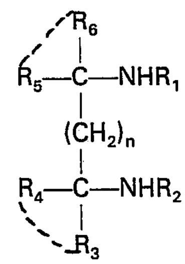

in der

$R_1$ und $R_2$ unabhängig voneinander ausgewählt sind aus Wasserstoff, Halogen, Alkyl mit 1 bis 12 Kohlenstoff-Atomen, Hydroxyalkyl, Halogenoalkyl, Cyanoalkyl, Aminoalkyl, Iminoalkyl, Alkyloxyalkylen, Nitro, Hydroxyalkyloxyalkylen, Cyanoalkyloxyalkylen, Alkenyl, Alkinyl und Carboalkoxy, wobei

$R_3$ bis $R_6$ unabhängig voneinander ausgewählt sind aus Alkyl mit 1 bis 12 Kohlenstoff-Atomen, Cycloalkyl, Hydroxycycloalkyl, Aminoalkyl, Iminoalkyl, Alkenyl und Aralkyl,

mit der weiteren Maßgabe, daß zwei beliebige der vorbezeichneten, an das gleiche Kohlenstoff-Atom gebundenen Substituenten Wasserstoff-Atome sein können oder gemeinsam einen cyclischen oder alicyclischen Kohlenwasserstoff bilden können, und

n eine Zahl von 0 bis 3 ist,

mit einem stöchiometrischen Überschuß von sowohl

(i) einem festen Ätzalkali und

(ii) einem $\alpha$-Trihalogenoalkylalkohol der Formeln

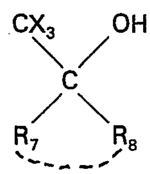

und

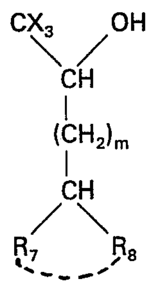

in denen

$R_7$ und $R_8$ gleich den im Vorstehenden definierten $R_3$ bis $R_6$ sind,

m 0 bis 4 ist und

X Halogen ist,

in Berührung gebracht wird.

2. Verfahren nach Anspruch 1, dadurch gekennzeichnet, daß die Temperatur der Reaktionsteilnehmer auf 10°C oder darunter gehalten wird.

3. Verfahren nach Anspruch 1, dadurch gekennzeichnet, daß der Phasen-Transfer-Katalysator ein Ammoniumsalz ist.

4. Verfahren nach Anspruch 1, dadurch gekennzeichnet, daß die Reaktionsteilnehmer und der Phasen-Transfer-Katalysator in einem unpolaren organischen Reaktionsmedium vereinigt werden.

5. Verfahren nach Anspruch 4, dadurch gekennzeichnet, daß das 1,4-Diaza-cycloalkan-2-on in dem unpolaren organischen Reaktionsmedium in hohem Maße löslich ist.

6. Verfahren nach Anspruch 1, dadurch gekennzeichnet, daß das Diamin N'-Isopropyl-2-methyl-1,2-propandiamin ist.

7. Verfahren nach Anspruch 1, dadurch gekennzeichnet, daß der Alkohol ein $\alpha$-Trichloroalkylalkohol ist.

8. Verfahren nach Anspruch 1, dadurch gekennzeichnet, daß die relative Stoffmengen-Konzentration ("Mol-Konzentration") des festen Ätzalkali zu dem Diamin in dem Bereich von 3,3:1 bis 6:1 liegt.

9. Verfahren nach Anspruch 1, dadurch gekennzeichnet, daß das feste Ätzalkali Natriumhydroxid ist.

10. Modifikation des Verfahrens von Anspruch 1, dadurch gekennzeichnet, daß anstelle von (ii) ein Gemisch aus einem Haloform und einem $\alpha$-Cyanoalkylalkohol der Formeln

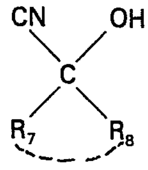 und 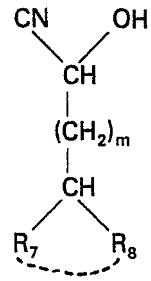

in denen
$R_7$ und $R_8$ gleich den im Vorstehenden definierten $R_3$ bis $R_6$ sind und
m 0 bis 4 ist,
eingesetzt wird.

11. Modifikation des Verfahrens von Anspruch 1, dadurch gekennzeichnet, daß anstelle von (ii) ein Gemisch aus einem Haloform und einem Keton der Formel

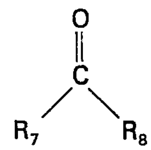

in der
$R_7$ und $R_8$ gleich den im Vorstehenden definierten $R_3$ bis $R_6$ sind,
eingesetzt wird.

**Revendications**

1. Procédé pour la synthèse de 1,4-diaza-cycloalcane-2-ones, caractérisé en ce qu'il consiste à mettre en contact, en présence d'un catalyseur de transfert de phase, lequel, en présence des réactifs suivants, provoque une condensation des co-réactifs et de la diamine de façon à conduire à la formation d'une cétone cyclique dans laquelle l'oxygène est doublement lié à un $\alpha$-carbone du produit co-réactif, une diamine de formule

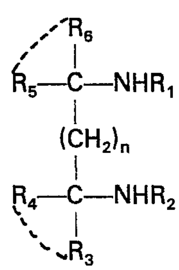

où $R_1$ et $R_2$ sont choisis, indépendamment l'un de l'autre, parmi l'hydrogène, un halogène, un alkyle à 1 à 12 atomes de carbone, un hydroxyalkyle, un halogénoalkyle, un cyanoalkyle, un aminoalkyle, un iminoalkyle, un alkyloxyalkylène, le nitro, un hydroxyalkyloxyalkylène, un cyanoalkyloxyalkylène, un alcényle, un alcynyle et un carbalcoxy; $R_3$ à $R_6$ étant choisis, indépendamment les uns des autres, parmi un alkyle à 1 à 12 atomes de carbone, un cycloalkyle, un hydroxycycloalkyle, un aminoalkyle, un iminoalkyle, un alcényle et un aralkyle, à la condition toutefois que deux quelconques des substituants ci-dessus pendant du même atome de carbone puissent être des atomes d'hydrogène ou former collectivement un hydrocarbure cyclique ou alicyclique, et n est un nombre compris entre 0 et 3; sur un excès stoechiométrique (i) de base caustique solide et (ii) d'un alcool $\alpha$-trihalogénoalkylique de formules

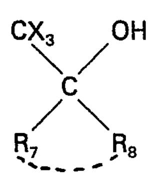 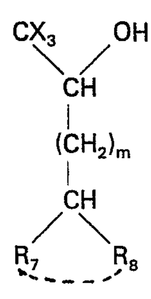

où $R_7$ et $R_8$ sont les mêmes que $R_3$—$R_6$ définis ci-dessus, m valant de 0 à 4 et X étant un halogène.

2. Procédé selon la revendication 1, dans lequel la température des réactifs est maintenue à 10°C ou moins.

3. Procédé selon la revendication 1, dans lequel le catalyseur de transfert de phase est un sel d'ammonium.

4. Procédé selon la revendication 1, dans lequel les réactifs et le catalyseur sont combinés dans un milieu de réaction organique non polaire.

5. Procédé selon la revendication 4, dans lequel la 1,4-diaza-cycloalcane-2-one est très soluble dans le milieu de réaction organique non polaire.

6. Procédé selon la revendication 1, dans lequel la diamine est la N'-isopropyl-2-méthyl-1,2-propanediamine.

7. Procédé selon la revendication 1, dans lequel l'alcool est un alcool $\alpha$-trichloralkylique.

8. Procédé selon la revendication 1, dans lequel la concentration molaire relative de la base caustique solide par rapport à la diamine est comprise entre 3,3:1 et 6:1.

9. Procédé selon la revendication 1, dans lequel la base caustique solide est de l'hydroxyde de sodium.

10. Modification du procédé selon la revendication 1, caractérisée en ce que l'on utilise à la place de (ii) un mélange d'halogénoforme et d'un alcool $\alpha$-cyanalkylique de formules:

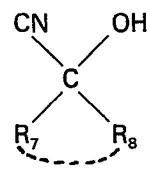 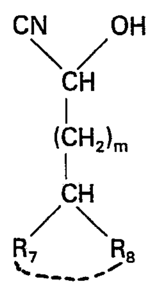

où $R_7$ et $R_8$ sont les mêmes que $R_3$—$R_6$ définis ci-dessus, et m vaut 0 à 4.

11. Modification du procédé selon la revendication 1, caractérisée en ce qu'on utilise à la place de (ii) un mélange d'un halogénoforme et d'une cétone aliphatique de formule

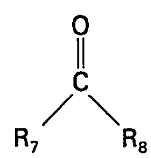

où $R_7$ et $R_8$ sont les mêmes que $R_3$—$R_6$ définis ci-dessus.